# NOUVEAU FASCICULE DE BREVET EUROPEEN

(11) **EP 1 148 863 B2**
(45) Date de publication et mention de la décision concernant l'opposition: **22.02.2012**
(45) Mention de la délivrance du brevet: 26.06.2002
(21) Numéro de dépôt: 00901717.9
(22) Date de dépôt: 04.02.2000
(51) Int. Cl.: A61Q 5/02, A61K 8/37, A61K 8/44, A61K 8/46, A61Q 5/12

(54) **COMPOSITIONS COSMETIQUES DETERGENTES ET UTILISATION**
REINIGENDE KOSMETISCHE ZUSAMMENSETZUNGEN UND IHRE VERWENDUNG
DETERGENT COSMETIC COMPOSITIONS AND USE

(30) Priorité: 05.02.1999 FR 9901386
(43) Date de publication de la demande: 31.10.2001
(73) Titulaire: L'Oréal, 75008 Paris (FR)
(72) Inventeur: RESTLE, Serge, F-95390 Saint-Prix (FR); GARNIER, Nathalie, Springfield, NJ 07081 (US)
(74) Mandataire: Dossmann, Gérard
(86) Numéro de dépôt international: PCT/FR2000/000271
(87) Numéro de publication internationale: WO 2000/045781

(56) Documents cités:
- EP-A- 0 511 652
- EP-A- 0 562 638
- WO-A-93/08787
- WO-A1-95//17163
- WO-A1-96//17590
- WO-A1-97//05857
- WO-A1-97//09031
- WO-A1-98//53039
- WO-A2-99//37744
- DE-A- 19 640 186
- DE-A1- 3 033 929
- DE-A1- 4 129 986
- DE-A1- 19 646 882
- JP-A- 61 155 310
- US-A- 4 426 310

## Description

La présente invention concerne de nouvelles compositions cosmétiques à propriétés améliorées destinées simultanément au nettoyage et au conditionnement des matières kératiniques telles que les cheveux, et comprenant, dans un support aqueux cosmétiquement acceptable, au moins un tensioactif anionique, au moins un tensioactif amphotère et au moins un ester d'acide carboxylique particulier, le rapport en poids tensioactif anionique /tensioactif amphotère étant inférieur ou égal à 3. L'invention concerne aussi l'utilisation desdites compositions dans l'application cosmétique susmentionnée.

Pour le nettoyage et/ou le lavage des matières kératiniques telles que les cheveux, l'utilisation de compositions détergentes (telles que les shampooings) à base essentiellement d'agents tensioactifs classiques de type notamment anionique, non ionique et/ou amphotère, mais plus particulièrement de type anionique, est courante. Ces compositions sont appliquées sur cheveux mouillés et la mousse générée par massage ou friction avec les mains permet, après rinçage à l'eau, l'élimination des diverses salissures initialement présentes sur les cheveux ou la peau.

Ces compositions de base possèdent certes un bon pouvoir lavant, mais les propriétés cosmétiques intrinsèques qui leur sont attachées restent toutefois assez faibles, notamment en raison du fait que le caractère relativement agressif d'un tel traitement de nettoyage peut entraîner à la longue sur la fibre capillaire des dommages plus ou moins marqués liés en particulier à l'élimination progressive des lipides ou protéines contenues dans ou à la surface de cette dernière.

Aussi, pour améliorer les propriétés cosmétiques des compositions détergentes ci-dessus, et plus particulièrement de celles qui sont appelées à être appliquées sur des cheveux sensibilisés (i.e. des cheveux qui se trouvent abîmés ou fragilisés notamment sous l'action chimique des agents atmosphériques et/ou de traitements capillaires tels que permanentes, teintures ou décolorations), il est maintenant usuel d'introduire dans ces dernières des agents cosmétiques complémentaires dits agents conditionneurs destinés principalement à réparer ou limiter les effets néfastes ou indésirables induits par les différents traitements ou agressions que subissent, de manière plus ou moins répétée, les fibres capillaires. Ces agents conditionneurs peuvent bien entendu également améliorer le comportement cosmétique des cheveux naturels.

Dans ce but, on a déjà proposé d'utiliser des agents conditionneurs insolubles. Ces composés insolubles présentent l'inconvénient d'être difficiles à maintenir en dispersion régulière dans le milieu.

Pour les maintenir en suspension, on a déjà proposé l'utilisation des dérivés d'esters ou d'éthers à longue chaîne (agents dispersants)(EP181773) ou des polysaccharides tels que la gomme de xanthane (agents gélifiants) (EP190010). Cependant, les agents dispersants présentent des problèmes de cristallisation qui entraînent parfois une évolution (augmentation) de la viscosité des compositions au cours du temps ; les agents gélifiants présentent également des inconvénients, à savoir d'une part que la mousse des compositions détergentes contenant des polysaccharides se développe difficilement (mauvais démarrage de mousse) et que, d'autre parts les compositions n'ont pas une texture lisse et s'écoulent par paquets, ce qui est peu apprécié des utilisateurs. De plus, ces divers agents ne permettent pas d'obtenir des compositions transparentes ou limpides.

La présente invention a pour but de proposer des compositions ne présentant pas les inconvénients des compositions citées ci-dessus.

Les agents conditionneurs doivent également être véhiculées sur les matières kératiniques traitées en vue de leur conférer, suivant l'application, des propriétés de douceur, de brillance et de démêlage.

EP0562638 décrit une composition aqueuse de shampooing contenant de l'eau, un tensio-actif anionique, un agent de conditionnement cationique, un agent de conditionnement insoluble dans l'eau de viscosité particulière tel que le myristate d'isopropyle et un agent de suspension. Le rapport tensioactif anionique/tensioactif amphotère n'est pas décrit.

EP0511652 décrit une composition aqueuse de shampooing contenant de l'eau, un tensio-actif anionique nettoyant, un polymère de conditionnement cationique, un tensioactif de conditionnement cationique et un ester gras.

DE19640186 décrit une composition de geldouche contenant de l'eau, un tensio-actif anionique , une bétaine, le paimitate d'isopropyle à 0,5% en poids.

WO93/08787 décrit une composition aqueuse de shampooing contenant de l'eau, un tensio-actif anionique, un polymère de conditionnement cationique hydrosoluble , une silicone insoluble, et un ester gras ayant plus de 10 atomes de carbone à moins de 1% en poids. De plus la composition contient un tensioactif cationique.

Ainsi, à la suite d'importantes recherches menées sur la question, il a maintenant été trouvé par la Demanderesse, qu'en utilisant une base lavante particulière, au moins un ester d'acide carboxylique particulier, il est possible d'obtenir des compositions détergentes stables présentant d'excellentes propriétés cosmétiques, en particulier le démêlage et le lissage des cheveux traités et ayant de bonnes propriétés d'usage tel qu'un bon pouvoir lavant intrinsèque et un bon pouvoir moussant.

La mise en oeuvre industrielle est extrêmement facile et les propriétés cosmétiques des shampooings sont excellentes.

Les compositions obtenues sont stables au stockage, sans nécessiter l'addition d'agent de dispersion et/ou de mise en suspension de l'ester selon l'invention.

En l'absence de composés additionnels insolubles, les compositions obtenues sont également transparentes. Elles peuvent contenir des quantités importantes d'ester d'acide carboxylique tout en conservant une bonne transparence et en ayant de bonnes propriétés cosmétiques.

Les compositions conformes à l'invention confèrent aux cheveux, notamment après rinçage, un remarquable effet traitant qui se manifeste notamment par une facilité de démêlage, ainsi qu'un apport de lissage, de douceur et de souplesse sans aucune sensation de gras.

Ainsi, la présente invention a pour objet de nouvelles compositions cosmétiques détergentes et conditionnantes caractérisée par le fait qu'elles comprennent, dans un milieu aqueux cosmétiquement acceptable, (A) une base lavante comprenant, au moins un tensioactif anionique et au moins un tensioactif amphotère présente à une teneur pondérale comprise entre 6 % et 35 % en poids par rapport au poids total de la composition, (B) au moins un ester d'acide carboxylique insoluble dans l'eau choisi parmi
1)- les monoesters de monoacides carboxyliques, linéaires, saturés ou insaturés et de monoalcools linéaires ou ramifiés, saturés ou insaturés, ou les monoesters de monoacides carboxyliques ramifiés, saturés ou insaturés et de monoalcools ramifiés, saturés ou insaturés
2)- les di ou tri esters de di ou triacides carboxyliques linéaires ou ramifiés, saturés ou insaturés et de monoalcools linéaires ou ramifiés, saturés ou insaturés,
3)- les mono, di ou triesters de di ou triacides carboxyliques linéaires ou ramifiés, saturés ou insaturés en C₁-C₄₈ et de dialcools linéaires ou ramifiés, saturés ou insaturés,
4)- les monoesters de mono acides carboxyliques linéaires ou ramifiés, saturés ou insaturés et de dialcools linéaires ou ramifiés, saturés ou insaturés,en C₃-C₃₀
5)- les diesters de monoacides carboxyliques linéaires ou ramifiés, saturés ou insaturés et de dialcools insaturés quelconques ou de dialcools saturés ayant plus de 4 atomes de carbone,
6)- les diesters de monoacides carboxyliques linéaires ou ramifiés, saturés ou insaturés et de trialcools saturés,
7)- les triesters de monoacides carboxyliques linéaires ou ramifiés, saturés ou insaturés et de trialcools saturés ayant plus de 3 atomes de carbone,
8)- les mono, di ou triesters de monoacides carboxyliques linéaires ou ramifiés, saturés ou insaturés et de trialcools insaturés,
9)- les mono, di ou triesters de di ou triacides carboxyliques linéaires ou ramifiés, saturés ou insaturés et de trialcools linéaires ou ramifiés, saturés ou insaturés, le nombre total d'atomes de carbone de l'ester ne dépassant pas 27 s'il ne contient pas d'insaturation et 50 s'il contient au moins une insaturation,
la concentration de l'ester étant comprise entre 1,2 et 15 % en poids par rapport au poids total de la composition,
la composition étant exempte de tensioactif cationique, et
le rapport en poids tensioactif anionique / tensioactif amphotère étant inférieur ou égal à 3.

La présence de tensioactifs cationiques dans les compositions selon l'invention diminue les performances cosmétiques desdites compositions.

L'invention a également pour objet l'utilisation en cosmétique des compositions ci-dessus pour le nettoyage et /ou le démaquillage et/ou le conditionnement des matières kératiniques telles que les cheveux et la peau.

Par exempte de tensioactif cationique, on comprend que la composition contient moins de 0,3% en poids de tensioactif cationique par rapport au poids total de la composition, de préférence moins de 0,1% en poids et plus particulièrement que la concentration en tensioactif cationique est nulle. Par tensioactif cationique, on ne désigne pas les polymères tensioactifs cationiques. Les polymères tensioactifs ne sont pas exclus de la composition.

### A- BASE LAVANTE :

La base lavante comprend un ou plusieurs tensioactifs anioniques et un ou plusieurs tensioactifs amphotères.

### (i) Tensioactif(s) anionique(s) :

Leur nature ne revêt pas, dans le cadre de la présente invention, de caractère véritablement critique.

Ainsi, à titre d'exemple de tensioactifs anioniques utilisables, seuls ou en mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 12 à 20 atomes de carbone, et le radical aryle désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'éthylène, et leurs mélanges.

On utilise de préférence un agent tensioactif anionique choisi parmi les alkyl(C₁₂-C₁₄) sulfates de sodium, de triéthanolamine ou d'ammonium, les alkyl (C₁₂-C₁₄)éthersulfates de sodium oxyéthylénés à 2,2 moles d'oxyde d'éthylène, le cocoyl iséthionate de sodium et l'alphaoléfine(C₁₄-C₁₆) sulfonate de sodium.

Parmi les tensioactifs anioniques, on préfère utiliser selon l'invention les sels d'alkylsulfates et d'alkyléthersulfates et leurs mélanges.

### (iii) Tensioactif(s) amphotère(s):

Les agents tensioactifs amphotères, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Parmi les dérivés d'amines, on peut citer les produits commercialisés sous les dénomination MIRANOL®, tels que décrits dans les brevets US-2 528 378 et US-2 781 354 et de structures :

R₂ -CONHCH₂CH₂ -N(R₃)(R₄)(CH₂COO-) (2)

dans laquelle : R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R_{2'}-CONHCH₂CH₂-N(B)(C) (3)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂-CHOH-SO₃H
R_{2'} désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL® C2M concentré par la société RHONE POULENC.

Selon la présente invention, on préfère plus particulièrement utiliser les agents tensio-actifs amphotères appartenant au groupe des bétaïnes tels que les alkylbétaïnes en particulier la cocoylbétaïne commercialisée sous la dénomination « DEHYTON AB 30 » en solution aqueuse à 30 % de MA par la société HENKEL ou les alkylamidobétaines telles que la TEGOBETAINE® F50 commercialisée par la société GOLDSCHMIDT.

La quantité minimale de base lavante est celle juste suffisante pour conférer à la composition finale un pouvoir moussant et/ou détergent satisfaisant, et des quantités trop importantes de base lavante n'apportent pas vraiment d'avantages supplémentaires.

Ainsi, selon l'invention, la base lavante peut représenter plus préférentiellement de 8 % à 25 % en poids, du poids total de la composition finale.

A titre indicatif, les compositions détergentes conformes à l'invention présentent généralement les compositions suivantes :
(i) tensio-actif(s) anionique(s) : de 3 à 30 % en poids, de préférence de 5 à 20 % en poids, par rapport au poids total de la composition détergente ;
(ii) tensio-actif(s) amphotère(s): de 1 à 20 % en poids, de préférence de 1,5 à 15 % en poids, par rapport au poids total de la composition.

Le rapport en poids tensioactif anionique / tensioactif amphotère est de préférence compris entre 0,2 et 3 plus particulièrement entre 0,4 et 2,5.

### B- Esters d'acide carboxylique

Les esters d'acide carboxylique insolubles dans l'eau selon l'invention sont insolubles dans l'eau à une concentration supérieure ou égale à 0,1% en poids dans l'eau à 25°C, c'est à dire qu'ils ne forment pas dans l'eau une solution isotrope transparente.

Les esters d'acide carboxylique insolubles dans l'eau selon l'invention peuvent contenir des groupements hydroxyles.

Les esters d'acide carboxylique selon l'invention sont généralement choisis parmi :
1)- les monoesters de monoacides carboxyliques linéaires, saturés ou insaturés en C₁-C₄₉ de préférence en C₃-C₃₀ et de monoalcools linéaires ou ramifiés, saturés ou insaturés en C₁-C₄₉ de préférence en C₂-C₃₀, les monoesters de monoacides ramifiés, saturés ou insaturés en C₁-C₄₉, de préférence en C₃-C₃₀, et de monoalcools ramifiés, saturés ou insaturés en C₁-C₄₉, de préférence en C₂-C₃₀.
   Parmi ces monoesters , on peut citer le lactate de cétyle ; le lactate d'alkyle en C₁₂-C₁₅ ; le lactate d'isostéaryle ; le lactate de lauryle ; le lactate de linoléyle ; le lactate d'oléyle ; l'octanoate de (iso)stéaryle ; l'octanoate d'isocétyle ; l'octanoate d'octyle ; l'octanoate de cétyle ; l'octanoate d'isodécyle ; l'isononanoate d'isononyle ; l'isononate de 2-éthylhexyle ; le palmitate d'octyle ; le pélargonate d'octyle ; le stéarate d'octyle ; l'érucate d'octyldodécyle ; l'érucate d'oléyle ; les palmitates d'éthyle et d'isopropyle, le palmitate d'éthyl-2-hexyle, le myristate d'isopropyle, de butyle, le stéarate d'hexyle, le stéarate de butyle, le stéarate d'isobutyle ; le laurate d'hexyle ; l'érucate de tridécyle.
2)- les di ou tri esters de di ou triacides carboxyliques linéaires ou ramifiés, saturés ou insaturés en C₂-C₄₈ de préférence en C₃-C₃₀ et de monoalcools linéaires ou ramifiés, saturés ou insaturés en C₁-C₄₉ de préférence en C₂-C₃₀ Parmi ces esters , on peut citer le sébacate de diéthyle ; le sébacate de diisopropyle ; l'adipate de diisopropyle ; l'adipate de di n-propyle ; l'adipate de dioctyle ; le maléate de dioctyle ; le citrate de triisopropyle ; le citrate de trioléyle ; le malate de dioctyle.
3)- les mono, di ou triesters de di ou triacides carboxyliques linéaires ou ramifiés, saturés ou insaturés en C₂-C₄₉ de préférence en C₃-C₃₀ et de dialcools linéaires ou ramifiés, saturés ou insaturés en C₁-C₄₉ de préférence en C₂-C₃₀.
4)- les monoesters de mono acides carboxyliques linéaires ou ramifiés, saturés ou insaturés en C₃-C₃₀ et de dialcools linéaires ou ramifiés, saturés ou insaturés en C₃-C₃₀
   Parmi ces esters , on peut citer le monostéarate de propylèneglycol, le monostéarate de tripropylèneglycol, le monostéarate de diéthylèneglycol, le monooléate de diéthylèneglycol.
5)- les diesters de monoacides carboxyliques linéaires ou ramifiés, saturés ou insaturés en C₁-C₄₆ de préférence en C₃-C₃₀ et de dialcools insaturés en C₂-C₄₈ de préférence en C₄-C₃₀ ou de dialcools saturés ayant plus de 4 atomes de carbone, et de préférence en C₅-C₄₈ et encore plus particulièrement en C₅-C₃₀
   Parmi ces esters , on peut citer le dilaurate de néopentylglycol, le dioctanoate de dipropylèneglycol, le dioctanoate de 2-butène 1,4-diol, le distéarate de 2-butène 1,4-diol
6)- les diesters de monoacides carboxyliques linéaires ou ramifiés, saturés ou insaturés en C₁-C₄₇ de préférence en C₃-C₃₀ et de trialcools saturés en C₃-C₄₉ de préférence en C₃-C₃₀
   Parmi ces esters, on peut citer le dilaurate de glycéryle, le dioléate de glycéryle.
7)- les triesters de monoacides carboxyliques linéaires ou ramifiés, saturés ou insaturés en C₁-C₄₆ de préférence en C₃-C₃₀ et de trialcools saturés ayant plus de 3 atomes de carbone et de préférence en C₄-C₄₇ et plus particulièrement en C₄-C₃₀
   Parmi ces esters , on peut citer le trihexanoate de triméthylolpropane, le tripentanoate de 1,2,6-hexanetriol.
8)- les mono, di ou triesters de monoacides carboxyliques linéaires ou ramifiés, saturés ou insaturés en C₁-C₄₇ de préférence en C₃-C₃₀ et de trialcools insaturés en C₃-C₄₉ de préférence en C₃-C₃₀
   Parmi ces esters , on peut citer le laurate de 2,5-diméthyl-3-hexyne-1,2,5-triol.
9)- les mono, di ou triesters de di ou triacides carboxyliques linéaires ou ramifiés, saturés ou insaturés en C₂-C₄₇ de préférence en C₃-C₃₀ et de trialcools linéaires ou ramifiés, saturés ou insaturés en C₃-C₄₈ de préférence en C₃-C₃₀
   Parmi ces esters , on peut citer le citrate de glycéryle, le monosuccinate de glycéryle.

Les esters d'acide carboxylique sont plus particulièrement choisis parmi les composés des classes 1), 2), 4), 6) et 9).

Le ou les esters d'acide carboxylique peuvent être utilisées dans les compositions conformes à l'invention dans des concentrations comprises de préférence entre 1,5 et 10 % en poids par rapport au poids total de la composition et encore plus particulièrement de 2 à 8% en poids.

Selon un mode de réalisation de l'invention, les compositions peuvent comprendre en outre un sel hydrosoluble et/ou un alcool hydrosoluble mono ou polyhydroxylé.

Selon un autre mode de réalisation de l'invention, les compositions comprenant les monoesters de monoacide carboxylique et de monoalcool dont le nombre total de carbone est supérieur ou égal à 24 et inférieur ou égal à 27, les mono et diesters de monoacides saturés et de glycérol dont le nombre total de carbone est supérieur ou égal à 17, les diesters de monoalcools ramifiés et de diacides insaturés comprennent généralement un sel hydrosoluble et/ou un alcool hydrosoluble mono ou polyhydroxylé.

Par hydrosolubles dans l'eau, on entend les composés solubles dans l'eau à une concentration supérieure ou égale à 0,1% en poids dans l'eau à 25°C, c'est à dire qu'ils forment une solution isotrope transparente.

Les sels hydrosolubles selon l'invention sont de préférence les sels de métaux mono ou divalents et d'un acide minéral ou organique.

On peut citer en particulier le chlorure de sodium, le chlorure de potassium, le chlorure de calcium, le sulfate de magnésium, le citrate de sodium, les sels de sodium de l'acide phosphorique. De préférence, on utilise les sels de métaux monovalents. Le chlorure de sodium est particulièrement préféré.

Les sels hydrosolubles sont présents généralement à des concentrations comprises entre 0,1 et 10% en poids et de préférence entre 0,5 et 5% en poids par rapport au poids total de la composition.

Les alcools hydrosolubles mono ou polyhydroxylés sont notamment les alcools inférieurs en C₁-C₆, comme l'éthanol, l'isopropanol, le tertiobutanol, le n-butanol, les polyols tels que les alkylèneglycols comme le propylèneglycol, la glycérine et les polyalkylèneglycols ; les éthers de glycols.

Le ou les alcools hydrosolubles peuvent être utilisés dans des concentrations généralement comprises entre 0,1 et 20% en poids et plus particulièrement entre 0,2 et 10% en poids par rapport au poids total de la composition.

Les compositions détergentes selon l'invention présentent un pH final généralement compris entre 3 et 8. De préférence, ce pH est compris entre 4 et 6,5. L'ajustement du pH à la valeur désirée peut se faire classiquement par ajout d'une base (organique ou minérale) dans la composition, par exemple de la soude, de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide minéral ou organique, de préférence l'acide citrique ou l'acide chlorhydrique.

Le milieu aqueux cosmétiquement acceptable peut être constitué uniquement par de l'eau ou par un mélange d'eau et d'un solvant cosmétiquement acceptable.

Les compositions conformes à l'invention peuvent contenir en plus de l'association définie ci-dessus des agents régulateurs de viscosité tels que des agents épaississants. On peut citer en particulier les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit commercialisé sous la dénomination "AMINOL A15" par la société CHEM Y, les acides polyacryliques réticulés et les copolymères acide acrylique / acrylates d'alkyle en C₁₀-C₃₀ réticulés. Ces agents régulateurs de viscosité sont utilisés dans les compositions selon l'invention dans des proportions pouvant aller jusqu'à 10 % en poids par rapport au poids total de la composition.

Les compositions conformes à l'invention peuvent également contenir jusqu'à 5 % d'agents nacrants ou opacifiants bien connus dans l'état de la technique tels que par exemple les alcools gras, les palmitates de sodium ou de magnésium, les stéarates et hydroxystéarates de sodium ou de magnésium, les alcool gras, les dérivés acylés à chaîne grasse tels que les distéarates d'éthylène glycol ou de polyéthylèneglycol, les éthers à chaînes grasses tels que par exemple le distéaryléther ou le 1-(hexadécyloxy)-2-octadécanol.

Les compositions conformes à l'invention peuvent éventuellement contenir en outre d'autres agents ayant pour effet d'améliorer les propriétés cosmétiques de cheveux ou de la peau sans cependant altérer la stabilité des compositions. On peut citer à ce sujet les polymères anioniques ou non ioniques ou cationiques ou amphotères, les protéines, les hydrolysats de protéines, les céramides, les pseudocéramides, les acides gras à chaînes linéaires ou ramifiées en C₁₆-C₄₀ tels que l'acide méthyl-18 eicosanoïque, les hydroxyacides, les vitamines, le panthénol, les silicones, volatiles ou non volatiles, solubles et insolubles dans le milieu, les filtres UV, les agents hydratants, les agents antipelliculaires ou antiséborrhéiques, les agents anti-radicaux libres, et leurs mélanges.

Selon un mode particulièrement préféré, les compositions selon l'invention comprennent en outre au moins un polymère cationique.

Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A-2 270 846, 2 383 660, 2 598 611, 2 470 596 et 2 519 863.

De manière encore plus générale, au sens de la présente invention, l'expression « polymère cationique » désigne tout polymère contenant des groupements cationiques et/ou des groupements ionisables en groupements cationiques.

Les polymères cationiques utilisables selon l'invention ont de préférence une densité de charge cationique supérieure ou égale à 0,2 meq./g, et plus particulièrement comprise entre 0,2 et 8,5 meq./g.

Parmi tous les polymères cationiques susceptibles d'être utilisés dans le cadre de la présente invention, on préfère mettre en oeuvre les dérivés d'éther de cellulose quaternaires tels que les produits commercialisés sous la dénomination « JR 400 » par la société UNION CARBIDE CORPORATION, les cyclopolymères, en particulier les homopolymères de sel de diallyldiméthylammonium et les copolymères de sel de diallyldiméthylammonium et d'acrylamide en particulier les chlorures, commercialisés sous les dénominations « MERQUAT 100 », « MERQUAT 550 » et « MERQUAT S » par la société MERCK, les polysaccharides cationiques et plus particulièrement les gommes de guar modifiées par du chlorure de 2,3-époxypropyl triméthylammonium commercialisées par exemple sous la dénomination « JAGUAR C13S » par la société MEYHALL, les homopolymères et les copolymères éventuellement réticulés de sel de (méth)acryloyloxyéthyltriméthylammonium, vendus par la société ALLIED COLLOIDS en solution à 50% dans de l'huile minérale sous les dénominations commerciales SALCARE SC92 (copolymère réticulé du chlorure de méthacryloyloxyéthyltriméthylammonium et de l'acrylamide) et SALCARE SC95 (homopolymère réticulé du chlorure de méthacryloyloxyéthyl triméthylammonium), les copolymères quaternaires de vinylpyrrolidone et de sel de vinyl imidazole tels que les produits commercialisés par BASF sous les dénominations LUVIQUAT FC 370, LUVIQUAT FC 550, LUVIQUAT FC 905 et LUVIQUAT HM-552.

On peut également utiliser les polymères qui sont constitués de motifs récurrents répondant à la formule : 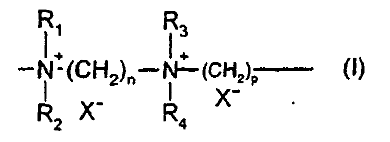 dans laquelle R₁, R₂, R₃ et R₄, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.

Un composé de formule (I) particulièrement préféré est celui pour lequel R₁, R₂, R₃ et R₄, représentent un radical méthyle et n = 3, p = 6 et X = Cl, dénommé Hexadimethrine chloride selon la nomenclature INCI (CTFA).

Selon l'invention, le ou les polymères cationiques peuvent représenter de 0,001 % à 10 % en poids, de préférence de 0,005 % à 5 % en poids, et encore plus préférentiellement de 0,01 % à 3 % en poids, du poids total de la composition finale.

Les compositions selon l'invention peuvent contenir également des synergistes de mousses tels que des 1,2-alcanediols en C₁₀-C₁₈ ou des alcanolamides gras dérivés de mono ou de diéthanolamine.

Bien entendu, l'homme du métier veillera à choisir ce ou ces éventuels composés complémentaires et/ou leurs quantités de manière telle que la solubilité des esters d'acide carboxylique selon l'invention, la stabilité des composition et les propriétés cosmétiques attachées intrinsèquement à la composition conforme à l'invention ne soient pas, ou substantiellement pas, altérées par la ou les adjonctions envisagées. L'addition de certains composés tels que les agents nacrants peut rendre la composition non transparente.

La transparence peut se mesurer par la turbidité au turbidimètre HACH - Modèle 2100 P à 25°C (L'appareil est étalonné avec de la formazine). La turbidité des compositions selon l'invention (en l'absence de composés additionnels insolubles) est alors généralement comprise entre 0,05 et 100 NTU et de préférence inférieure à 50 NTU. Lorsque l'ester selon l'invention est sous forme de particules dispersées, la taille de ces particules est de préférence inférieure à 5 nanomètres.

Le pouvoir moussant des compositions selon l'invention, caractérisé par une hauteur de mousse, est généralement supérieur à 75 mm ; de préférence, supérieure à 100 mm mesurée selon la méthode ROSS-MILES (NF T 73-404 /ISO696) modifiée.
Les modifications de la méthode sont les suivantes :
La mesure se fait à la température de 22°C avec de l'eau osmosée. La concentration de la solution est de 2g/l. La hauteur de la chute est de 1m. La quantité de composition qui chute est de 200 ml. Ces 200 ml de composition tombe dans une éprouvette ayant un diamètre de 50 mm et contenant 50 ml de la composition à tester. La mesure est faite 5 minutes après l'arrêt de l'écoulement de la composition.

Ces compositions peuvent se présenter sous la forme de liquides plus ou moins épaissis, de crèmes ou de gels et elles conviennent principalement au lavage, au soin des matières kératiniques en particulier des cheveux et de la peau et encore plus particulièrement des cheveux.

L'invention a également pour objet un procédé de lavage et de conditionnement des matières kératiniques telles que notamment les cheveux consistant à appliquer sur lesdites matières mouillées une quantité efficace d'une composition telle que définie ci-dessus, puis à effectuer un rinçage à l'eau après un éventuel temps de pause.

Les compositions selon l'invention sont utilisées de préférence comme shampooings pour le lavage et le conditionnement des cheveux et ils sont appliqués dans ce cas-là sur les cheveux humides dans des quantités efficaces pour les laver, et la mousse générée par massage ou friction avec les mains est ensuite éliminée après un éventuel temps de pause, par rinçage à l'eau, l'opération pouvant être répétée une ou plusieurs fois.

Les compositions conformes à l'invention sont également utilisables comme gels douche pour le lavage et le conditionnement des cheveux et/ou de la peau, auquel cas ils sont appliqués sur la peau et/ou les cheveux humides et sont rincés après application.

Des exemples concrets, mais nullement limitatifs, illustrant l'invention vont maintenant être donnés.

### EXEMPLE 1

On a réalisé quatre compositions de shampooings conformes à l'invention

| | 1 | 2 | 3 | 4 |
|---|---|---|---|---|
| - Lauryléthersulfate de sodium (C12/C14 à 70/30) à 2,2 moles d'oxyde d'éthylène en solution aqueuse à 70% de MA | 15 gMA | 15 gMA | 5,25 gMA | 15 gMA |
| - Cocoylbétaïne à 30% MA(DEHYTON AB 30) | 5 gMA | 5 gMA | 9 gMA | 5 gMA |
| - Palmitate d'isopropyle | 2 g | | | |
| - Néopentanoate d'isodécyle | | 2g | | 6 g |
| - Myristate d'isopropyle | | | 2 g | |
| - Homopolymère de chlorure de diallyl diméthyl ammonium en solution aqueuse à 40% de MA (MERQUAT 100 de CALGON) | 0,4 gMA | 0,4 gMA | 0,4 gMA | 0,4 gMA |
| - NaCl | 4g | 4g | 4g | 4g |
| - Parfum, conservateur | qs | qs | qs | qs |
| - Acide chlorhydrique qs pH | 6,2 | 6,3 | 6,8 | 6,3 |
| - Eau déminéralisée qs | 100 g | 100 g | 100 g | 100 g |
| | | | | |
| Turbidité (NTU) | 41,7 | 11,9 | 8,5 | 7 |

Les compositions 1 à 4 selon l'invention sont transparentes et stables. (La transparence est évaluée par turbidimétrie en unités NTU (Nephelometric turbidity units))

Les cheveux traités avec ces compositions se démêlent facilement et sont lisses de la racine à la pointe.

### EXEMPLE 2

On a réalisé quatre compositions de shampooings, conformes à l'invention

| | 5 | 6 | 7 | 8 | 9 |
|---|---|---|---|---|---|
| - Lauryléthersulfate de sodium (C12/C14 à 70/30) à 2,2 moles d'oxyde d'éthylène en solution aqueuse à 70% de MA (MA = matière active) | 15 gMA | 15 gMA | 10 gMA | 15 gMA | 15 gMA |
| - Cocoylbétaïne à 30% MA(DEHYTON AB 30) | 5 gMA | 5 gMA | 10 gMA | 5 gMA | 5 gMA |
| - Ricinoléate d'octyldodécyle | | | | 1,2 g | |
| - Lactate de myristyle | | 2g | | | |
| - Myristate d'isopropyle | 2 g | | 6 g | | |
| - Di(linoléate de diisopropyle) | | | | | 1,2 g |
| - Polyquaternium-10 (JR 400 de UNION CARBIDE) | 0,4 gMA | 0,4 gMA | | 0,4 gMA | |
| - NaCl | | | | | 4 g |
| - Parfum, conservateur | qs | qs | qs | qs | qs |
| - Acide chlorhydrique qs pH | 6,1 | 6,1 | 6,1 | 6,5 | 6,5 |
| - Eau déminéralisée qs | 100 g | 100 g | 100 g | 100 g | 100 g |
| | | | | | |
| Turbidité (NTU) | 2,9 | 4,2 | 2,9 | 6,5 | 8,2 |

Les compositions 5 à 8 selon l'invention sont transparentes et stables.

Les cheveux traités avec ces compositions se démêlent facilement et sont lisses de la racine à la pointe.

## Revendications

1. Composition cosmétique détergentes et conditionnants, **caracterisée par le fait qu'**elle comprend, dans un milieu aqueux cosmétiquement acceptable (A) une base lavante comprenant au moins un tensioactif anionique et sur moins un tensioactif amphotère, présente à une teneur pondérale comprise entre 6% et 35% en poids par rapport au poids total de la composition,
(B) au moins en ester d'acide carboxylique insoluble dans l'eau choisi parmi
1)- les monoesters de monoacides carboxyliques linéaires ou ramifiés, saturés ou insaturés et de monoalcools linéaires est ramifiés, saturés ou insaturés, ou, les monoesters de monoacides carboxyliques linéaires ou ramifiés, saturés ou insaturés et de monoalcools linéaires ou ramifiés, saturés ou insaturés,
2)- les di ou tri esters de di ou triacides carboxiliques linéaires ou ramifiés, saturés ou insaturés et de monoalcools linéaires ou ramifiés, saturés ou insaturés,
3)- les mono, di ou triesters de di ou triacides carboxiliques linéaires ou ramifiés, saturés ou insaturés et de dialcools linéaires ou ramifiés, saturés ou insaturés,
4)- les monoesters de mono acides carboxiliques linéaires ou ramifiés, saturés ou insaturés en C₁-C₄₀ et de dialcools linéaires ou ramifiés, saturés ou insaturés en C₃-C₃₀
5)- les diesters de monoacides carboxyliques linéaires ou ramifiés, saturés ou insaturés et de dialcools insaturés quelconque ou de dialcools saturés ayant plus de 4 atomes de carbone,
6)- les diesters de monoacides carboxyliques linéaires ou ramifiés, saturés ou insaturés et de trialcools saturés,
7)- les triesters de monoacides carboxyliques linéaires ou ramifiés, saturés ou insaturés et de trialcools saturés ayant plus de 3 atomes de carbone,
8)- les mono, di ou triesters de monoacides carboxyliques linéaires ou ramifiés, saturés ou insaturés et de trialcools insaturés,
9)- les mono, di ou triesters de di ou triacides carboxyliques linéaires ou ramifiés, saturés ou insaturés et de trialcools linéaires ou ramifiés, saturés ou insaturés,
le nombre total d'atomes de carbone de l'ester ne dépassant pas 27 s'il ne contient pas d'insaturation et 50 s'il contient au moins une insaturation,
la concentration de l'ester étant comprise entre 1,2 et 15% en poids par rapport au poids total de la composition
la composition étant exampte de tensioactif cationique, et
le rapport en poids tensioactif anionique / tensioactif amphotère étant inférieur ou égal à 3.

2. Composition selon la revendication 1, **caractérisée par le fait que** ladite base lavante est présente à une teneur pondérale comprise entre 8 % et 25 % en poids.

3. Composition selon l'une quelconque des revendications 1 ou 2, **caractérisée en ce que** le(s) tensioactif(s) anionique(s) est (sont) présent(s) dans les concentrations allant de 3 à 30% en poids, de préférence de 5 à 20% en poids, par rapport au poids total de la composition.

4. Composition selon l'une quelconque des revendications 1 à 3, **caractérisée en ce que** le(s) tensioactifs(s) amphotère(s) est(sont) présent(s) dans des concentrations allant de 1 à 20 % en poids, de préférence de 1,5 à 15% en poids, par rapport au poids total de la composition.

5. Composition selon l'une quelconque des revendications 1 à 4, **caractérisée par le fait que** le rapport en poids tensioactif anionique / tensioactifs amphotère est compris entre 0,2 et 3 et plus particulièrement entre 0,4 et 2,5.

6. Composition selon l'une quelconque des revendications 1 à 5, **caractérisée par le fait que** lesdits esters sont choisis parmi :
1)- les monoesters de monoacides carboxyliques linéaires saturés ou insaturés en C1-C48 de préférence en C3-C30 et de monoalcools linéaires ou ramifiés, saturés ou insaturés en C1-C49 de préférence un C2-C30, ou
les monoesters de monoacides carboxyliques ramifiés, saturés ou insaturés en C1-C49 de préférence en C3-C30 et de monoalcools ramifiés, saturés ou insaturés en C1-C49 de préférence en C2-C30,
2)- les di ou tri esters de di ou triacides carboxyliques linéaires ou ramifiés, saturés ou insaturés en C₂₋C₄₈ de préférence en C₂-C₃₀ et de monoalcools linéaires ou ramifiés, saturés ou insaturés en C₁-C₄₈ de préférence en C₃-C₃₀
3)- les mono, di ou triesters de di ou triacides carboxyliques linéaires ou ramifiés, saturés ou insaturés en C₂-C₄₈ de préférence en C₃-C₃₀ et de dialcools linéaires ou ramifiés, saturés ou insaturés en C₁-C₄₉ de préférence en C₂-C₃₀
4)- les monoesters de mono acides carboxyliques linéaires ou ramifiés, saturés ou insaturés en C₃-C₃₀ et de dialcools linéaires ou ramifiés, saturés ou insaturés en C₃-C₃₀
5)- les diesters de monoacides carboxyliques linéaires ou ramifiés, saturés ou insaturés en C₁-C₄₆ de préférence en C₃-C₃₀ et de dialcools insaturés en C₂-C₄₈ de préférence en C₄-C₃₀ ou de dialcools saturés ayant plus de 4 atomes de carbone, et de préférence en C₅-C₄₈ et encore plus particulièrement en C₅-C₃₀
6)- les diesters de monoacides carboxyliques linéaires ou ramifiés, saturés ou insaturés en C₁-C₄₇ de préférence en C₃-C₃₀ et de trialcools saturés en C₃-C₄₉ de préférence en C₃-C₃₀
7)- les triesters de monoacides carboxyliques linéaires ou ramifiés, saturés ou insaturés en C₁-C₄₆ de préférence en C₃-C₃₀ et de trialcools saturés ayant plus de 3 atomes de carbone et de préférence en C₄-C₄₇ et plus particulièrement en C₄-C₃₀
8)- les mono, di ou triesters de monoacides carboxyliques linéaires ou ramifiés, saturés ou insaturés en C₁-C₄₇ de préférence en C₃-C₃₀ et de trialcools insaturés en C₃-C₄₉ de préférence en C₃-C₃₀
9)- les mono, di ou triesters de di ou triacides carboxyliques linéaires ou ramifiés, saturés ou insaturés en C₂-C₄₇ de préférence en C₃-C₃₀ et de trialcools linéaires ou ramifiés, saturés ou insaturés en C₃-C₄₈ de préférence en C₃-C₃₀

7. Composition selon la revendication 6, **caractérisée par le fait que** lesdits esters sont choisis parmi composés des classes 1), 2), 4), 6) et 9).

8. Composition selon l'une quelconque des revendications 6 ou 7, **caractérisée par le fait que** lesdits esters sont choisis parmi :
le lactate de cétyle ; le lactate d'alkyle en C₁₂-C₁₅ ; le lactate d'isostéaryle ; le lactate de tauryle ; le lactate de linoléyle ; le lactate d'oléyle ; l'octanoate de (iso)stéaryle ; l'octanoate d'isocétyle ; l'octanoate d'octyle ; l'octanoate de cétyle ; l'octanoate d'isodécyle ; l'isononanoate d'isononyle ; l'isononate de 2-éthylhexyle; la palmitate d'octyle; le pélargonate d'octyle ; le stéarate d'octyle; l'érucate d'octyldodécyle ; l'érucate d'oléyle ; les palmitates d'éthyle et d'isopropyle, le palmitate d'éthyl-2-hexyle, le myristate d'isopropyle, de butyle, le stéarate d'hexyle, le stéarate de butyl, le stéarate d'isobutyle ; le laurate d'hexyle : l'érucate de tridécyle,
le sebacate de diéthyle ; le sébacate de disopropyle ; l'adipate de diisopropyle : l'adipate de di n-propyle ; l'adipate de dioctyle ; le maléate de dioctyle; le citrate de triisopropyle : le citrate de trioléyle ; le malate de dioctyle.
le monostéarate de propylèneglycol, le monostéarate de tripropylèneglycol, le monostéarate de diéthylèneglycol, le monooléate de diéthylèneglycol, le dilaurate de glycéryle, le dioléate de glycéryle,
le citrate de glycéryle, le monosuccinate de glycéryle.

9. Composition selon l'une quelconque des revendications 1 à 8, **caracterisée par le fait que** lesdits esters sont présents dans des concentrations comprises entre 1,5 et 10 % en poids par rapport au poids total de la composition et encore plus particulièrement de 2 à 8% en poids.

10. Composition selon l'une quelconque des revendications 1 à 9, **caractérisée par le fait que** la composition comprend en outre au moins un polymère cationique.

11. Composition selon la revendication 10, **caractérisée par le fait que** le polymère cationique est choisi parmi les dérivés d'éther de cellulose quaternaires, les homopolymères de sel de diallyldiméthylammonium et les copolymères de sel de diallyldiméthylammonium et d'acrylamide, les polysaccharides cationiques, les copolymères quaternaires de vinylpyrrolidone et de sel de vinyl imidazole.

12. Composition selon la revendication 10, **caractérisée par le fait que** le polymère cationique est choisi parmi les polymères qui sont constitués de motifs récurrents répondant à la formule: 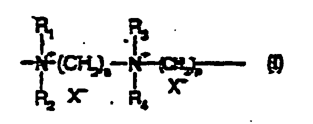 dans laquelle R₁, R₂, R₃ et R₄ identiques ou différents, désignant un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.

13. Composition selon l'une quelconque revendication 12 à 12, **caractérisée en ce que** ledit polymère cationique représente de 0,001 % à 10 % en poids, de préférence de 0,005% à 5 % en poids, et encore plus préférentiellement de 0,01 % à 3 % en poids, du poids total de la composition.

14. Composition selon l'une quelconque du revendications 1 à 13, **caractérisée par le fait que** la composition comprend en outre au moins un sel hydrosoluble.

15. Composition selon la revendication 14, **caractérisée par le fait que** les sels hydrosolubles sont des sels de métaux mono ou divalents et d'un acide minéral ou organique

16. Composition selon l'une quelconque des revendications 14 ou 15, **caractérisée par le fait que** les sels hydrosolubles sont choisis parmi le chlorure de sodium, le chlorure de potassium, le chlorure de calcium, le sulfate de magnésium, le citrate de sodium, les sels de sodium de l'acide phosphorique.

17. Composition selon l'une quelconque des revendications 14 à 16, **caractérisée par le fait que** les sels hydrosolubles sont présents à des concentrations comprises entre 0,1 et 10% en poids et de préférence entre 0,5 et 5% en poids par rapport au poids total de la composition.

18. Composition selon l'une quelconque des revendications 1 à 17, **caractérisée par le fait que** la composition comprend en outre au moins un alcool hydrosoluble.

19. Composition selon la revendication 18, **caractérisée par le fait que** les alcools hydrosolubles sont les alcools inférieurs en C₁-C₆, comme l'éthanol, l'isopropanol, le tertiobutanol, le n-butanol, les polyols tels que les alkylèneglycols comme le propylèneglycol, la glycérine et les polyalkylèneglycols ; les éthers de glycols.

20. Composition selon l'une quelconque des revendications 18 ou 19, **caractérisée en ce que** les alcools hydrosolubles sont utilisés dans des concentrations généralement comprises entre 0,1 et 20% en poids et plus particulièrement entre 0,2 et 10% en poids par rapport au poids total de la composition.

21. Composition selon l'une quelconque des revendications 1 à 20, **caractérisée par le fait qu'**elle contient en plus un ou plusieurs adjuvants choisis parmi les polymères anioniques ou non ioniques ou amphotères, les protéines, les hydrolysats de protéines, les céramides, les pseudocéramides, les acides gras à chaînes linéaires ou ramifiées en C₁₆-C₄₀ tels que l'acide méthyl-18 eicosanoïque, les hydroxyacides, les vitamines, le panthénol, les silicones volatiles ou non volatiles, solubles ou insolubles dans le milieu, les filtres UV, les agents hydratants, les agents antipelliculaires ou antiséborrhéiques, les agents anti-radicaux libres, les agents opacifiants et leurs mélanges.

22. Utilisation d'une composition telle que définie dans l'une quelconque des revendications 1 à 21 pour le nettoyage et/ou le démaquillage des matières kératiniques.

23. Procédé cosmétique de lavage et de conditionnement des matières kératiniques telles que les cheveux consistant à appliquer sur lesdites matières mouillées une quantité efficace d'une composition telle que définie dans l'une quelconque des revendications 1 à 21, puis à effectuer un rinçage à l'eau après un éventuel temps de pause.

## Claims

1. Detergent and conditioning cosmetic composition, **characterized in that** it comprises, in a cosmetically acceptable aqueous medium, (A) a washing base comprising at least one anionic surfactant and at least one amphoteric surfactant, present at a content by weight of between 6% and 35% by weight with respect to the total weight of the composition, (B) at least one water-insoluble carboxylic acid ester chosen from
1)- monoesters of saturated or unsaturated and linear monocarboxylic acids and of saturated or unsaturated and linear or branched monoalcohols, or monoesters of saturated or unsaturated and branched monocarboxylic acids and of saturated or unsaturated and branched monoalcohols,
2)- di- or triesters of saturated or unsaturated and linear or branched di- or tricarboxylic acids and of saturated or unsaturated and linear or branched monoalcohols,
3)- mono-, di- or triesters of saturated or unsaturated and linear or branched di- or tricarboxylic acids and of saturated or unsaturated and linear or branched dialcohols,
4)- monoesters of saturated or unsaturated and linear or branched C₁-C₄₈ monocarboxylic acids and of saturated or unsaturated and linear or branched C₃-C₃₀ dialcohols,
5)- diesters of saturated or unsaturated and linear or branched monocarboxylic acids and of unsaturated dialcohols of any kind or of saturated dialcohols having more than 4 carbon atoms,
6)- diesters of saturated or unsaturated and linear or branched monocarboxylic acids and of saturated trialcohols,
7)- triesters of saturated or unsaturated and linear or branched monocarboxylic acids and of saturated trialcohols having more than 3 carbon atoms,
8)- mono-, di- or triesters of saturated or unsaturated and linear or branched monocarboxylic acids and of unsaturated trialcohols,
9)- mono-, di- or triesters of saturated or unsaturated and linear or branched di- or tricarboxylic acids and of saturated or unsaturated and linear or branched trialcohols,
the total number of carbon atoms of the ester not exceeding 27 if it is not unsaturated and 50 if it comprises at least one unsaturation,
the concentration of the ester being between 1.2 and 15% by weight with respect to the total weight of the composition,
the composition being devoid of cationic surfactant, and
the anionic surfactant/amphoteric surfactant ratio by weight being less than or equal to 3.

2. Composition according to Claim 1, **characterized in that** the said washing base is present at a content by weight of between 8% and 25% by weight.

3. Composition according to either one of Claims 1 and 2, **characterized in that** the anionic surfactant(s) is (are) present in concentrations ranging from 3 to 30% by weight, preferably from 5 to 20% by weight, with respect to the total weight of the composition.

4. Composition according to any one of Claims 1 to 3, **characterized in that** the amphoteric surfactant(s) is (are) present in concentrations ranging from 1 to 20% by weight, preferably from 1.5 to 15% by weight, with respect to the total weight of the composition.

5. Composition according to any one of Claims 1 to 4, **characterized in that** the anionic surfactant/amphoteric surfactant ratio by weight is between 0.2 and 3 and more particularly between 0.4 and 2.5.

6. Composition according to any one of Claims 1 to 5, **characterized in that** the said esters are chosen from:
1)- monoesters of saturated or unsaturated and linear C₁-C₄₉, preferably C₃-C₃₀, monocarboxylic acids and of saturated or unsaturated and linear or branched C₁-C₄₉, preferably C₂-C₃₀, monoalcohols, or monoesters of saturated or unsaturated and branched C₁-C₄₉, preferably C₃-C₃₀, monocarboxylic acids and of saturated or unsaturated and branched C₁-C₄₉, preferably C₂-C₃₀, monoalcohols,
2)- di- or triesters of saturated or unsaturated and linear or branched C₂-C₄₈, preferably C₃-C₃₀, di- or tricarboxylic acids and of saturated or unsaturated and linear or branched C₁-C₄₉, preferably C₂-C₃₀, monoalcohols,
3)- mono-, di- or triesters of saturated or unsaturated and linear or branched C₂-C₄₉, preferably C₃-C₃₀, di- or tricarboxylic acids and of saturated or unsaturated and linear or branched C₁-C₄₉, preferably C₃-C₃₀, dialcohols,
4)- monoesters of saturated or unsaturated and linear or branched C₃-C₃₀ monocarboxylic acids and of saturated or unsaturated and linear or branched C₃-C₃₀ dialcohols,
5)- diesters of saturated or unsaturated and linear or branched C₁-C₄₆, preferably C₃-C₃₀, monocarboxylic acids and of unsaturated C₂-C₄₈, preferably C₄-C₃₀, dialcohols or of saturated dialcohols having more than 4 carbon atoms and preferably C₅-C₄₈ dialcohols and more particularly still C₅-C₃₀ dialcohols,
6)- diesters of saturated or unsaturated and linear or branched C₁-C₄₇, preferably C₃-C₃₀, monocarboxylic acids and of saturated C₃-C₄₉, preferably C₃-C₃₀, trialcohols,
7)- triesters of saturated or unsaturated and linear or branched C₁-C₄₆, preferably C₃-C₃₀, monocarboxylic acids and of saturated trialcohols having more than 3 carbon atoms and preferably C₄-C₄₇, trialcohols and more particularly C₄-C₃₀ trialcohols,
8)- mono-, di- or triesters of saturated or unsaturated and linear or branched C₁-C₄₇, preferably C₃-C₃₀, monocarboxylic acids and of unsaturated C₃-C₄₉, preferably C₃-C₃₀, trialcohols,
9)- mono-, di- or triesters of saturated or unsaturated and linear or branched C₂-C₄₇, preferably C₃-C₃₀, di- or tricarboxylic acids and of saturated or unsaturated and linear or branched C₃-C₄₈, preferably C₃-C₃₀, trialcohols.

7. Composition according to Claim 6, **characterized in that** the said esters are chosen from the compounds from classes 1), 2), 4), 6) and 9).

8. Composition according to either one of Claims 6 and 7, **characterized in that** the said esters are chosen from:
cetyl lactate, C₁₂-C₁₅ alkyl lactate, isostearyl lactate, lauryl lactate, linoleyl lactate, oleyl lactate, (iso)stearyl octanoate, isocetyl octanoate, octyl octanoate, cetyl octanoate, isodecyl octanoate, isononyl isononanoate, 2-ethylhexyl isononate, octyl palmitate, octyl pelargonate, octyl stearate, octyldodecyl erucate, oleyl erucate, ethyl and isopropyl palmitates, 2-ethylhexyl palmitate, isopropyl myristate, butyl myristate, hexyl stearate, butyl stearate, isobutyl stearate, hexyl laurate or tridecyl erucate,
diethyl sebacate, diisopropyl sebacate, diisopropyl adipate, di(n-propyl) adipate, dioctyl adipate, dioctyl maleate, triisopropyl citrate, trioleyl citrate or dioctyl malate, propylene glycol monostearate, tripropylene glycol monostearate, diethylene glycol monostearate or diethylene glycol monooleate,
glyceryl dilaurate or glyceryl dioleate,
glyceryl citrate or glyceryl monosuccinate.

9. Composition according to any one of Claims 1 to 8, **characterized in that** the said esters are present in concentrations of between 1.5 and 10% by weight with respect to the total weight of the composition and more particularly still from 2 to 8% by weight.

10. Composition according to any one of Claims 1 to 9, **characterized in that** the composition additionally comprises at least one cationic polymer.

11. Composition according to Claim 10, **characterized in that** the cationic polymer is chosen from quaternary derivatives of cellulose ether, diallyldimethylammonium salt homopolymers and copolymers of diallyldimethylammonium salt and of acrylamide, cationic polysaccharides, or quaternary copolymers of vinylpyrrolidone and of vinylimidazole salt.

12. Composition according to Claim 10, **characterized in that** the cationic polymer is chosen from polymers which are composed of repeat units corresponding to the formula: 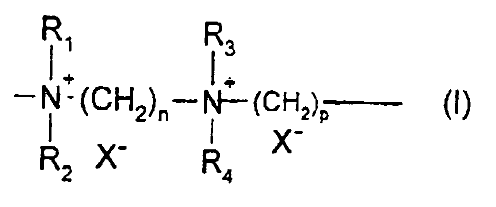 in which R₁, R₂, R₃ and R₄, which are identical or different, denote an alkyl or hydroxyalkyl radical having from 1 to 4 carbon atoms approximately, n and p are integers varying from 2 to 20 approximately and X-is an anion derived from an inorganic or organic acid.

13. Composition according to any one of Claims 10 to 12, **characterized in that** the said cationic polymer represents from 0.001% to 10% by weight, preferably from 0.005% to 5% by weight and more preferably still from 0.01% to 3% by weight of the total weight of the composition.

14. Composition according to any one of Claims 1 to 13, **characterized in that** the composition additionally comprises at least one water-soluble salt.

15. Composition according to Claim 14, **characterized in that** the water-soluble salts are salts of mono- or divalent metals and of an inorganic or organic acid.

16. Composition according to either one of Claims 14 and 15, **characterized in that** the water-soluble salts are chosen from sodium chloride, potassium chloride, calcium chloride, magnesium sulphate, sodium citrate, or the sodium salts of phosphoric acid.

17. Composition according to any one of Claims 14 to 16, **characterized in that** the water-soluble salts are present at concentrations of between 0.1 and 10% by weight and preferably between 0.5 and 5% by weight with respect to the total weight of the composition.

18. Composition according to any one of Claims 1 to 17, **characterized in that** the composition additionally comprises at least one water-soluble alcohol.

19. Composition according to Claim 18, **characterized in that** the water-soluble alcohols are lower C₁-C₆ alcohols, such as ethanol, isopropanol, tert-butanol or n-butanol, polyols, such as alkylene glycols, for example propylene glycol or glycerol, and polyalkylene glycols, or glycol ethers.

20. Composition according to either one of Claims 18 and 19, **characterized in that** the water-soluble alcohols are used in concentrations generally of between 0.1 and 20% by weight and more particularly between 0.2 and 10% by weight with respect to the total weight of the composition.

21. Composition according to any one of Claims 1 to 20, **characterized in that** it additionally comprises one or more adjuvants chosen from anionic or non-ionic or amphoteric polymers, proteins, protein hydrolysates, ceramides, pseudoceramides, fatty acids comprising linear or branched C₁₆-C₄₀ chains, such as 18-methyleicosanoic acid, hydroxy acids, vitamins, panthenol, volatile or non-volatile silicones which are soluble or insoluble in the medium, UV screening agents, moisturizing agents, antidandruff or antiseborrhoeic agents, agents for combating free radicals, opacifying agents, and their mixtures.

22. Use of a composition as defined in any one of Claims 1 to 21 for cleaning and/or removing make-up from keratinous substances.

23. Cosmetic process for washing and for conditioning keratinous substances, such as the hair, which consists in applying, to the said wetted substances, an effective amount of the composition as defined in any one of Claims 1 to 21 and in then rinsing with water, after an optional leave-in time.

## Patentansprüche

1. Reinigende und konditionierende kosmetische Zusammensetzung, **dadurch gekennzeichnet, daß** sie in einem kosmetisch akzeptablen wässrigen Medium (A) eine reinigende Basisformulierung, die mindestens einen anionischen grenzflächenaktiven Stoff und mindestens einen amphoteren grenzflächenaktiven Stoff enthält und die in einem Gewichtsanteil von 6 bis 35 Gew.-%, bezogen auf das Gesamtgewicht der Zusammensetzung vorliegt,
(B) mindestens einen in Wasser unlöslichen Carbonsäureester enthält, der ausgewählt ist unter:
1) Monoestern von geradkettigen, gesättigten oder ungesättigten Monocarbonsäuren und geradkettigen oder verzweigten, gesättigten oder ungesättigten Monoalkoholen oder
Monoestern von verzweigten, gesättigten oder ungesättigten Monocarbonsäuren und verzweigten, gesättigten oder ungesättigten Monoalkoholen;
2) Di- oder Triestern von geradkettigen oder verzweigten, gesättigten oder ungesättigten Di-oder Tricarbonsäuren und geradkettigen oder verzweigten, gesättigten oder ungesättigten Monoalkoholen;
3) Mono-, Di- oder Triestern von geradkettigen oder verzweigten, gesättigten oder ungesättigten Di- oder Tricarbonsäuren und geradkettigen oder verzweigten, gesättigten oder ungesättigten Dialkoholen;
4) Monoestern von geradkettigen oder verzweigten, gesättigten oder ungesättigten Monocarbonsäuren mit 1 bis 48 Kohlenstoffatomen und geradkettigen oder verzweigten, gesättigten oder ungesättigten Dialkoholen mit 3 bis 30 Kohlenstoffatomen;
5) Diestern von geradkettigen oder verzweigten, gesättigten oder ungesättigten Monocarbonsäuren und beliebigen ungesättigten Dialkoholen oder gesättigten Dialkoholen mit mehr als 4 Kohlenstoffatomen;
6) Diestern von geradkettigen oder verzweigten, gesättigten oder ungesättigten Monocarbonsäuren und gesättigten Trialkoholen;
7) Triestern von geradkettigen oder verzweigten, gesättigten oder ungesättigten Monocarbonsäuren und gesättigten Trialkoholen mit mehr als 3 Kohlenstoffatomen;
8) Mono-, Di- oder Triestern von geradkettigen oder verzweigten, gesättigten oder ungesättigten Monocarbonsäuren und ungesättigten Trialkoholen;
9) Mono-, Di- oder Triestern von geradkettigen oder verzweigten, gesättigten oder ungesättigten Di- oder Tricarbonsäuren und geradkettigen oder verzweigten, gesättigten oder ungesättigten Trialkoholen;
wobei die Gesamtzahl der Kohlenstoffatome des Esters 27 nicht übersteigt, wenn er keine ungesättigte Bindung aufweist, und 50 nicht übersteigt, falls er mindestens eine ungesättigte Bindung enthält;
wobei die Konzentration des Esters im Bereich von 1, 2 bis 15 Gew.-%, bezogen auf das Gesamtgewicht der Zusammensetzung, liegt,
die Zusammensetzung keine kationischen grenzflächenaktiven Stoffe enthält und
das Gewichtsverhältnis anionischer grenzflächenaktiver Stoff/amphoterer grenzflächenaktiver Stoff 3 ist oder darunter liegt.

2. Zusammensetzung nach Anspruch 1, **dadurch gekennzeichnet, daß** die reinigende Basisformulierung in einem Gewichtsanteil von 8 bis 25 Gew.-% vorliegt.

3. Zusammensetzung nach einem der Ansprüche 1 oder 2, **dadurch gekennzeichnet, daß** der (die) anionische(n) grenzflächenaktive(n) Stoff(e) in Konzentrationen von 3 bis 30 Gew.-% und vorzugsweise 5 bis 20 Gew.-%, bezogen auf das Gesamtgewicht der Zusammensetzung, vorliegen.

4. Zusammensetzung nach einem der Ansprüche 1 bis 3, **dadurch gekennzeichnet, daß** der (die) amphotere(n) grenzflächenaktive(n) Stoff(e) in Konzentrationen von 1 bis 20 Gew.-% und vorzugsweise 1,5 bis 15 Gew.-%, bezogen auf das Gesamtgewicht der Zusammensetzung, vorliegen.

5. Zusammensetzung nach einem der Ansprüche 1 bis 4, **dadurch gekennzeichnet, daß** das Gewichtsverhältnis anionisches Tensid/amphoteres Tensid im Bereich von 0,2 bis 3 und insbesondere 0,4 bis 2,5 liegt.

6. Zusammensetzung nach einem der Ansprüche 1 bis 5, **dadurch gekennzeichnet, daß** die Ester ausgewählt sind unter:
1) Monoestern von geradkettigen, gesättigten oder ungesättigten Monocarbonsäuren mit 1 bis 49 und vorzugsweise 3 bis 30 Kohlenstoffatomen und geradkettigen oder verzweigten, gesättigten oder ungesättigten Monoalkoholen mit 1 bis 49 und vorzugsweise 2 bis 30 Kohlenstoffatomen, oder Monoestern von verzweigten, gesättigten oder ungesättigten Monocarbonsäuren mit 1 bis 49 und vorzugsweise 3 bis 30 Kohlenstoffatomen und verzweigten, gesättigten oder ungesättigten Monoalkoholen mit 1 bis 49 und vorzugsweise 2 bis 30 Kohlenstoffatomen;
2) Di- oder Triestern von geradkettigen oder verzweigten, gesättigten oder ungesättigten Di-oder Tricarbonsäuren mit 2 bis 48 und vorzugsweise 3 bis 30 Kohlenstoffatomen und geradkettigen oder verzweigten, gesättigten oder ungesättigten Monoalkoholen mit 1 bis 49 und vorzugsweise 2 bis 30 Kohlenstoffatomen;
3) Mono-, Di- oder Triestern von geradkettigen oder verzweigten, gesättigten oder ungesättigten Di- oder Tricarbonsäuren mit 2 bis 49 und vorzugsweise 3 bis 30 Kohlenstoffatomen und geradkettigen oder verzweigten, gesättigten oder ungesättigten Dialkoholen mit 1 bis 49 und vorzugsweise 2 bis 30 Kohlenstoffatomen;
4) Monoestern von geradkettigen oder verzweigten, gesättigten oder ungesättigten Monocarbonsäuren mit 3 bis 30 Kohlenstoffatomen und geradkettigen oder verzweigten, gesättigten oder ungesättigten Dialkoholen mit 3 bis 30 Kohlenstoffatomen;
5) Diestern von geradkettigen oder verzweigten, gesättigten oder ungesättigten Monocarbonsäuren mit 1 bis 46 und vorzugsweise 3 bis 30 Kohlenstoffatomen und ungesättigten Dialkoholen mit 2 bis 48 und vorzugsweise 4 bis 30 Kohlenstoffatomen oder gesättigten Dialkoholen mit mehr als 4 Kohlenstoffatomen und vorzugsweise mit 5 bis 48 und insbesondere 5 bis 30 Kohlenstoffatomen;
6) Diestern von geradkettigen oder verzweigten, gesättigten oder ungesättigten Monocarbonsäuren mit 1 bis 47 und vorzugsweise 3 bis 30 Kohlenstoffatomen und gesättigten Trialkoholen mit 3 bis 49 und vorzugsweise 3 bis 30 Kohlenstoffatomen;
7) Triestern von geradkettigen oder verzweigten, gesättigten oder ungesättigten Monocarbonsäuren mit 1 bis 46 und vorzugsweise 3 bis 30 Kohlenstoffatomen und gesättigten Trialkoholen mit mehr als 3 Kohlenstoffatomen und vorzugsweise 4 bis 47 und insbesondere 4 bis 30 Kohlenstoffatomen;
8) Mono-, Di- oder Triestern von geradkettigen oder verzweigten, gesättigten oder ungesättigten Monocarbonsäuren mit 1 bis 47 und vorzugsweise 3 bis 30 Kohlenstoffatomen und ungesättigten Trialkoholen mit 3 bis 49 und vorzugsweise 3 bis 30 Kohlenstoffatomen;
9) Mono-, Di- oder Triestern von geradkettigen oder verzweigten, gesättigten oder ungesättigten Di- oder Tricarbonsäuren mit 2 bis 47 und vorzugsweise 3 bis 30 Kohlenstoffatomen und geradkettigen oder verzweigten, gesättigten oder ungesättigten Trialkoholen mit 3 bis 48 und vorzugsweise 3 bis 30 Kohlenstoffatomen.

7. Zusammensetzung nach Anspruch 6, **dadurch gekennzeichnet, daß** die Ester unter den Verbindungen der Gruppen 1), 2), 4), 6) und 9) ausgewählt sind.

8. Zusammensetzung nach einem der Ansprüche 6 oder 7, **dadurch gekennzeichnet, daß** die Ester ausgewählt sind unter:
Cetyllactat; C₁₂₋₁₅-Alkyllactat; Isostearyllactat; Lauryllactat; Linoleyllactat; Oleyllactat; (Iso)stearyloctanoat; Isocetyloctanoat; Octyloctanoat; Cetyloctanoat; Isodecyloctanoat; Isononylisononanoat; 2-Ethylhexylisononat; Octylpalmitat; Octylperlargonat; Octylstearat; Octyldodecylerucat; Oleylerucat; Ethylpalmitat; Isopropylpalmitat; 2-Ethylhexylpalmitat; Isopropylmyristat, Butylmyristat; Hexylstearat; Butylstearat; Isobutylstearat; Hexyllaurat; Tridecylerucat;
Diethylsebacat; Diisopropylsebacat; Diisopropyladipat; Di-n-Propyladipat; Dioctyladipat; Dioctylmaleat; Triisopropylcitrat; Trioleylcitrat; Dioctylmalat;
Propylenglykolmonostearat, Tripropylenglykolmonostearat, Diethylenglykolmonostearat; Diethylenglykolmonooleat, Glyceryldilaurat, Glyceryldioleat;
Glycerylcitrat und Glycerylmonosuccinat.

9. Zusammensetzung nach einem der Ansprüche 1 bis 8, **dadurch gekennzeichnet, daß** die Ester in Konzentrationen von 1,5 bis 10 Gew.-%, bezogen auf das Gesamtgewicht der Zusammensetzung, und insbesondere 2 bis 8 Gew.-% vorliegen.

10. Zusammensetzung nach einem der Ansprüche 1 bis 9, **dadurch gekennzeichnet, daß** sie ferner mindestens ein kationisches Polymer enthält.

11. Zusammensetzung nach Anspruch 10, **dadurch gekennzeichnet, daß** das kationische Polymer unter den quartären Celluloseetherderivaten, Homopolymeren von einem Diallyldimethylammoniumsalz und Copolymeren von einem Diallyldimethylammoniumsalz und Acrylamid, kationischen Polysacchariden und quaternisierten Copolymeren von Vinylpyrrolidon und einem Vinylimidazolsalz ausgewählt sind.

12. Zusammensetzung nach Anspruch 10, **dadurch gekennzeichnet, daß** das kationische Polymer unter den Polymeren ausgewählt ist, die aus wiederkehrenden Einheiten der folgenden Formel bestehen: 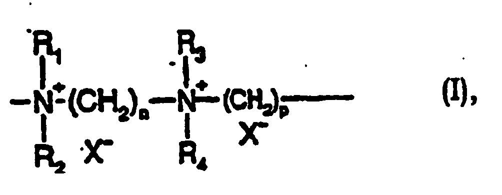 wobei die Gruppen R₁, R₂, R₃ und R₄, die identisch oder voneinander verschieden sind, eine Alkyl- oder Hydroxyalkylgruppe mit etwa 1 bis 4 Kohlenstoffatomen bedeuten, n und p ganze Zahlen im Bereich von etwa 2 bis 20 sind, und X⁻ ein von einer anorganischen oder organischen Säure abgeleitetes Anion ist.

13. Zusammensetzung nach einem der Ansprüche 10 bis 12, **dadurch gekennzeichnet, daß** das kationische Polymer 0,001 bis 10 Gew.-%, vorzugsweise 0,005 bis 5 Gew.-% und noch bevorzugter 0,01 bis 3 Gew.-%, bezogen auf das Gesamtgewicht der Zusammensetzung, ausmacht.

14. Zusammensetzung nach einem der Ansprüche 1 bis 13, **dadurch gekennzeichnet, daß** die Zusammensetzung ferner mindestens ein wasserlösliches Salz enthält.

15. Zusammensetzung nach Anspruch 14, **dadurch gekennzeichnet, daß** die wasserlöslichen Salze unter den Salzen von ein- oder zweiwertigen Metallen und einer anorganischen oder organischen Säure ausgewählt sind.

16. Zusammensetzung nach einem der Ansprüche 14 oder 15, **dadurch gekennzeichnet, daß** die wasserlöslichen Salze unter Natriumchlorid, Kaliumchlorid, Calciumchlorid, Magnesiumsulfat, Natriumcitrat und den Natriumsalzen von Phosphorsäure ausgewählt sind.

17. Zusammensetzung nach einem der Ansprüche 14 bis 16, **dadurch gekennzeichnet, daß** die wasserlöslichen Salze in Konzentrationen von 0,1 bis 10 Gew.-% und vorzugsweise 0,5 bis 5 Gew.-%, bezogen auf das Gesamtgewicht der Zusammensetzung, vorliegen.

18. Zusammensetzung nach einem der Ansprüche 1 bis 17, **dadurch gekennzeichnet, daß** die Zusammensetzung ferner mindestens einen wasserlöslichen Alkohol enthält.

19. Zusammensetzung nach Anspruch 18, **dadurch gekennzeichnet, daß** die wasserlöslichen Alkohole unter den niederen C₁₋₆-Alkoholen, wie Ethanol, Isopropanol, t-Butanol und n-Butanol, und Polyolen, wie Alkylenglykolen, beispielsweise Propylenglykol, Glycerin und Polyalkylenglykolen; und Glykolethern ausgewählt sind.

20. Zusammensetzung nach einem der Ansprüche 18 oder 19, **dadurch gekennzeichnet, daß** die wasserlöslichen Alkohole in Konzentrationen im allgemeinen im Bereich von 0,1 bis 20 Gew.-% und insbesondere 0,2 bis 10 Gew.-%, bezogen auf das Gesamtgewicht der Zusammensetzung, verwendet werden.

21. Zusammensetzung nach einem der Ansprüche 1 bis 20, **dadurch gekennzeichnet, daß** sie außerdem einen oder mehrere Zusatzstoffe enthält, die unter den anionischen, nichtionischen oder amphoteren Polymeren, Proteinen, Proteinhydrolysaten, Ceramiden, Pseudoceramiden, Fettsäuren mit linearen oder verzweigten C₁₆₋₄₀-Fettketten, wie 18-Methyleicosansäure, Hydroxysäuren, Vitaminen, Panthenol, flüchtigen oder nicht flüchtigen Siliconen, die in dem Medium löslich oder unlöslich sind, UV-Filtern, Hydratisierungsmitteln, Wirkstoffen gegen Schuppen oder gegen Seborrhö, Radikalfängern für freie Radikale, Trübungsmitteln und deren Gemischen ausgewählt sind.

22. Verwendung einer Zusammensetzung nach einem der Ansprüche 1 bis 21 zur Reinigung und/oder zum Abschminken von Keratinsubstanzen.

23. Kosmetisches Verfahren zur Reinigung und zum Konditionieren von Keratinsubstanzen, beispielsweise Haaren, das darin besteht, auf die feuchten Keratinsubstanzen eine Zusammensetzung nach einem der Ansprüche 1 bis 21 in einer wirksamen Menge aufzutragen und dann, gegebenenfalls nach einer Einwirkzeit, mit Wasser zu spülen.
